# EUROPÄISCHE PATENTSCHRIFT

(11) **EP 1 712 249 B1**
(45) Veröffentlichungstag und Bekanntmachung des Hinweises auf die Patenterteilung: **27.02.2008**
(21) Anmeldenummer: 06007579.3
(22) Anmeldetag: 11.04.2006
(51) Int. Cl.: A61M 25/02

(54) **Fixiervorrichtung zum Befestigen eines in eine Körperöffnung einbringbaren medizinischen Hilfsmittels**
Fixing device for the attachment of a medical device into an orifice of the body
Dispositif de fixation pour attachement d'un dispositif médical dans un orifice corporel

(30) Priorität: 12.04.2005 AT 6132005
(43) Veröffentlichungstag der Anmeldung: 18.10.2006
(73) Patentinhaber: Lang, Leonhard, 6020 Innsbruck (AT)
(72) Erfinder: Roediger, Claudia, 30938 Thönse (DE); Roediger, Frank, 30900 Wedemark (DE); Ohnmacht, Roland, 6020 Innsbruck (AT)
(74) Vertreter: Torggler, Paul Norbert

(56) Entgegenhaltungen:
- WO-A-94/28962
- WO-A-98/32481
- WO-A-20/05025664
- GB-A- 934 342
- US-A- 5 735 272
- US-A1- 2002 143 296

## Beschreibung

Die Erfindung betrifft eine Fixiervorrichtung zum Befestigen eines in eine Körperöffnung einbringbaren medizinischen Hilfsmittels, insbesondere durch die Nase einbringbare Beatmungstuben, an der Haut eines Subjektes, mit einem Trägerelement, das wenigstens einen ersten dem Subjekt zuordenbaren und einen zweiten dem Hilfsmittel zuordenbaren Abschnitt aufweist, die zumindest bereichsweise einseitig klebend ausgebildet sind.

Eine Gruppe der bisher bekannten, gattungsgemäßen Fixiervorrichtungen besteht im Wesentlichen aus einem elastischen Band, das an seinen Enden miteinander verbindbar ist. Dieses elastische Band wird dabei um einen Körperteil und die Fixiervorrichtung gelegt, um die Fixiervorrichtung in ihrer gewünschten Lage zu positionieren. Die Befestigung des elastischen Bandes am Körperteil erfolgt dabei in der Regel mittels handelsüblicher Pflaster. Die Praxis hat gezeigt, dass eine derartige Positionierung der Fixiervorrichtung nicht in erwünschtem Ausmaß stabil ist, wodurch es zu Komplikationen infolge einer Dislokation der Fixiervorrichtung oder des vollständigen Herausrutschens des Hilfsmittels aus der Körperöffnung kommen kann.

Weiter sind Fixiervorrichtungen mit einem Basisteil zum Befestigen der Fixiervorrichtung am Subjekt und einem oder mehreren am Basisteil angeordneten Fixierzügel zum Fixieren des Hilfsmittels bekannt. Diese bekannten Fixiervorrichtungen sind einseitig mit einer Klebeschicht versehen und werden mit dem Basisteil in der Nähe der Körperöffnung am Subjekt angeklebt. Nach dem Einbringen des medizinischen Hilfsmittels in die Körperöffnung wird das Hilfsmittel mit Hilfe der Fixierzügel positioniert. Als nachteilig dabei hat sich herausgestellt, dass mit einer derartigen Fixiervorrichtung dem medizinischen Hilfsmittel keine bevorzugte Richtung gegeben werden kann, wodurch es durch die zum Teil recht lange Tragedauer der medizinischen Hilfsmittel zu Reizungen und Entzündungen in den Bereichen der Körperöffnungen kommen kann, die durch den Druck des medizinischen Hilfsmittels in Mitleidenschaft gezogen werden.

US 5735272 A, WO 98/32481 A, US 2002/143296 A1, GB 934342 A und WO 94/28962 A offenbaren Fixiervorrichtungen mit normal zur Längsachse angeordneten und in Längsrichtung des Grundkörpers gegenüberliegenden Fixiermitteln.

Die vorliegende Erfindung hat es sich daher zur Aufgabe gemacht, eine Fixiervorrichtung zum Befestigen eines in eine Körperöffnung einbringbaren medizinischen Hilfsmittels so auszugestalten, dass die Fixiervorrichtung unter Vermeidung der genannten Nachteile einfach, flexibel und schnell angewendet werden kann, um die einzubringenden medizinischen Hilfsmittel sicher in einer korrekten Lage zu fixieren und deren Bewegungsfreiheit zu vermindern.

Erfindungsgemäß wird diese Aufgabe mit einer Fixiervorrichtung gelöst, bei der der zweite dem medizinischen Hilfsmittel zuordenbare Abschnitt einen länglichen Grundkörper und wenigstens zwei in Bezug auf die Längsachse des Grundkörpers gegenüberliegende, im Wesentlichen normal zur Längsachse angeordnete und in Längsrichtung des Grundskörpers voneinander beabstandete Fixiermittel aufweist.

Die Befestigung des zweiten Abschnittes der Fixiervorrichtung am medizinischen Hilfsmittel kann in einfacher Weise über ein Klebemittel erfolgen, wobei gemäß einer Ausführungsform der Erfindung die wenigstens zwei Fixiermittel zumindest bereichsweise einseitig klebend ausgebildet sind. Selbstverständlich ist es auch möglich, den länglichen Grundkörper des zweiten Abschnittes zumindest bereichsweise oder vollflächig einseitig klebend auszubilden.

Dadurch, dass der dem Hilfsmittel zuordenbare Abschnitt der Fixiervorrichtung zwei einseitig klebende Fixiermittel aufweist, wird eine Lagestabilisierung des medizinischen Hilfsmittels durch das Umschlingen des Hilfsmittels mit den abstehenden Fixiermitteln erreicht.

Gemäß einer weiteren Ausführungsform der Erfindung ist vorgesehen, dass der erste dem Subjekt zuordenbare Abschnitt des Trägerelementes wenigstens zwei voneinander unabhängig am Subjekt befestigbare Fixierelemente aufweist, wobei es sich als günstig herausgestellt hat, wenn der erste Abschnitt v-förmig ausgebildet bzw. die Fixierelemente des ersten Abschnittes v-förmig angeordnet sind.

Durch die erfindungsgemäß Ausbildung des dem Subjekt zuordenbaren Abschnittes der Fixiervorrichtung ergeben sich zwei voneinander unabhängige Klebepunkte an der Hautoberfläche, die beispielsweise bei der Verwendung der erfindungsgemäßen Vorrichtung zum Fixieren einer über die Nase eingeführten Sonde am rechten und linken Nasenflügel angebracht werden. Die v-förmige Ausbildung des ersten Abschnittes bedingt, dass wenigstens zwei Fixierelemente einen Winkel einschließen, der zwischen 30° und 180°, beispielsweise bei ca. 105° liegen kann. Der Winkel zwischen den beiden Fixierelementen spielt dabei eine untergeordnete Rolle, es kommt vielmehr darauf an, dass die wenigstens zwei Fixierelemente im Gebrauchszustand der Fixiervorrichtung in Bezug auf das medizinische Hilfsmittel auf zwei gegenüberliegenden Seiten am Subjekt befestigbar sind, wodurch dem medizinischen Hilfsmittel eine bevorzugte Richtung gegeben werden kann.

Gemäß einem weiteren Ausführungsbeispiel der Erfindung lässt sich die neuartige Fixiervorrichtung dann in einfacher Weise an der Haut eines Subjektes befestigen, wenn wenigstens eines der beiden Fixierelemente, vorzugsweise beide Fixierelemente, einen im Wesentlichen länglichen Grundkörper und einen gegenüber dem Grundktiper verbreiterten, vorzugsweise kreisflächenförmig oder ellipsenförmig ausgebildeten Endabschnitt aufweist und die Fixierelemente zumindest bereichsweise einseitig klebend ausgebildet sind.

Eine weitere Ausführungsform der Erfindung sieht vor, die Fixiervorrichtung wenigstens ein mit dem Trägerelement vorzugsweise unlösbar verbundenes Versteifungselement aufweist, wobei eine herstellungstechnisch einfache Lösung vorsieht, dass das wenigstens eine Versteifungselement auf der der klebenden Seite gegenüberliegenden Seite des Trägerelementes, das Trägerelement vorzugsweise teilweise abdeckend, angeordnet ist.

Durch das Versteifungselement wird also die Fixiereinrichtung insgesamt verstärkt, sodass eine sichere Fixierung des medizinischen Hilfsmittels auf Zug- und Druckbelastung gewährleistet ist.

Gemäß einem weiteren Ausführungsbeispiel der Erfindung weist das Trägerelement einen ersten dem Patienten zuordenbaren Abschnitt und einen zweiten dem Hilfsmittel zuordenbaren Abschnitt auf, wobei das Verstärkungselement den ersten und den zweiten Abschnitt zumindest teilweise überdeckend auf dem Trägerelement angeordnet ist. Im Wesentlichen erstreckt sich das Versteifungselement also von einem Ende des Trägerelementes bis zum anderen.

Wenn auch das Trägerelement bevorzugter Weise einteilig ausgebildet ist, sieht ein weiteres Ausführungsbeispiel der Erfindung vor, das Trägerelement zweiteilig auszubilden, sodass der eine dem Patienten zuordenbare Teil des Trägerelementes mit dem anderen dem medizinischen Hilfsmittel zuordenbaren Teil des Trägerelementes über das Versteifungselement verbunden ist.

Eine konstruktiv einfache Lösung der Anordnung des Versteifungselementes auf dem Trägerelement ergibt sich gemäß einem weiteren Ausführungsbeispiel dann, wenn das Versteifungselement auf das Trägerelement geklebt ist, vorzugsweise mittels eines doppelseitigen Klebebandes.

Um ein Loslösen des Versteifungselementes vom Trägerelement sicher zu verhindern, ist gemäß einer weiteren Ausführungsform der Erfindung vorgesehen, dass auf der der klebenden Seite gegenüber liegenden Seite des Trägerelementes eine Abdeckung angeordnet ist, wobei das Versteifungselement zwischen dem Trägerelement und der Abdeckung eingeschlossen, vorzugsweise einlaminiert, ist.

Wird dabei die Abdeckung von einer einseitig klebenden, vorzugsweise transparenten, PET-Folie gebildet, ist es nicht mehr notwendig, das Versteifungselement auf das Trägerelement aufzukleben, da in diesem Fall das Versteifungselement mittels der einseitig klebenden Abdeckung auf dem Trägerelement fixiert wird.

Gemäß einem besonders bevorzugten Ausführungsbeispiel der Erfindung ist vorgesehen, dass das vorzugsweise streifenförmige Versteifungselement im Wesentlichen entlang der Mittellängsachse des Trägerelementes an diesem angeordnet ist.

Obwohl es grundsätzlich denkbar wäre, das Trägerelement und das Versteifungselement aus demselben Material herzustellen, sieht ein weiteres Ausführungsbeispiel der Erfindung vor, das Trägerelement und das Versteifungselement aus unterschiedlichen Materialien herzustellen, wobei es sich als günstig erwiesen hat, wenn das Versteifungselement von einer zwischen 100 µm und 300 µm. vorzugsweise etwa 150 µm und 250 µm, dicken PE-Folie gebildet ist, wobei es sich als besonders günstig herausgestellt hat, wenn die PE-Folie etwa 190 µm dick ist.

Als besonders günstig für einen sicheren Halt der Fixiervorrichtung sowohl am medizinischen Hilfsmittel als auch an der Haut eines Subjektes hat es sich herausgestellt, wenn das Trägerelement einstückig und einseitig, vorzugsweise vollflächig, klebend ausgebildet ist Eine kostengünstige Lösung ergibt sich dabei, wenn das Trägerelement von einer Selbstklebefolie, vorzugsweise einer einseitig klebenden Polymer-Folie, gebildet ist.

Gemäß einem weiteren Ausführungsbeispiel der Erfindung kann die Ausbildung der klebenden Seite des Trägerelementes dadurch erfolgen, dass das Trägerelement von einer transparenten, selbstklebenden PET-Folie gebildet ist.

Um eine Verklebung der klebenden Bereiche des Trägerelementes bis zum Gebrauch zu verhindern, sieht eine weitere Ausführungsform der Erfindung vor, dass zumindest die klebenden Bereiche des Trägerelementes mit einer vorzugsweise silikonisierten Abdeckung versehen sind.

Weitere Vorteile der Erfindung und der durch sie erzielten Vorteile ergeben sich aus der nachstehenden Erläuterung des in der Zeichnung dargestellten Ausführungsbeispieles einer erfindungsgemäßen Fixiervorrichtung. Darin zeigt:

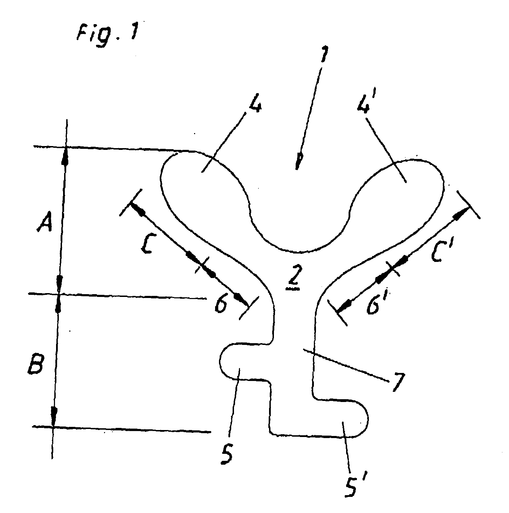

- Fig. 1: eine Draufsicht auf ein erstes Ausführungsbeispiel der Erfindung,
- Fig. 2a und 2b: eine Draufsicht und eine Seitenansicht auf das Ausführungsbeispiel nach Fig. 1 mit einer Abdeckung für die klebenden Bereiche und
- Fig. 3a und 3b: unterschiedliche perspektivische Ansichten des Ausführungsbeispieles im Gebrauchszustand,
- Fig. 4a: eine Draufsicht auf ein weiteres Ausführungsbeispiel der Erfindung,
- Fig. 4b: einen Querschnitt durch das in Fig. 4a gezeigte Ausführungsbeispiel entlang der Linie AA',
- Fig. 5a: eine Draufsicht auf ein weiteres Ausführungsbeispiel der Erfindung,
- Fig. 5b: einen Querschnitt durch das in Fig. 5a gezeigte Ausführungsbeispiel entlang der Linie BB',
- Fig. 6a: eine Draufsicht auf ein weiteres Ausführungsbeispiel der Erfindung,
- Fig.6b: einen Querschnitt durch das in Fig. 6a dargestellte Ausführungsbeispiel entlang der Linie CC' und
- Fig. 7: ein Anwendungsbeispiel der Erfindung zur Fixierung einer über die Nase eingeführten Sonde.

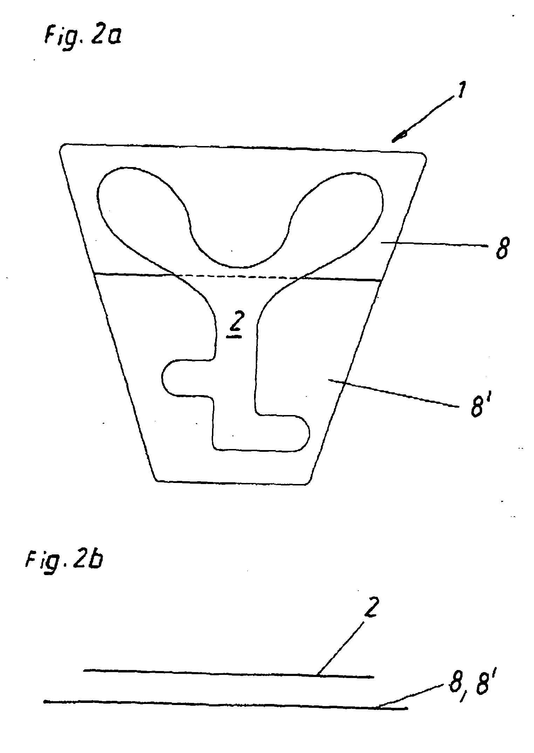

Beim dargestellten Ausführungsbeispiel weist die erfindungsgemäße Fixiervorrichtung 1 ein Trägerelement 2 auf, das einen ersten dem Subjekt zuordenbaren Abschnitt A und einen zweiten dem medizinischen Hilfsmittel zuordenbaren Abschnitt B umfasst. Das Trägerelement 2 wird von einer einseitig klebenden Polymer-Folie gebildet, wobei die klebende Seite der Polymer-Folie bis zum Gebrauch der Fixiervorrichtung 1 mit einer silikonisierten Abdeckung 8,8' bedeckt ist (Fig. 2a, 2b).

Die silikonisierte Abdeckung 8, 8' ist größer als das Trägerelement 2 und zweiteilig ausgebildet, um ein einfaches Ablösen der Abdeckung 8, 8' sicherzustellen.

Der erste, dem Subjekt zuordenbare Abschnitt A weist erfindungsgemäß zwei voneinander unabhängig am Subjekt befestigbare Fixierelemente 4, 4' auf, von denen jedes einen im Wesentlichen länglichen Grundkörper 5, 5' und einen gegenüber dem Grundkörper 5, 5' verbreiterten ellipsenförmig ausgebildeten Endabschnitt C, C' aufweist.

Dadurch, dass die beiden Fixierelemente 4, 4' des ersten Abschnittes A v-förmig angeordnet sind und der längliche Grundkörper 7 des zweiten Abschnittes B im Wesentlichen in der Verlängerung der Winkelhalbierenden der beiden Fixierelemente 4, 4' verläuft, ergeben sich an der Haut des Subjektes 10 zwei Klebepunkte, die in Bezug auf den länglichen Grundkörper 7 des zweiten Abschnittes B auf gegenüberliegenden Seiten liegen, wodurch es möglich ist, dem medizinischen Hilfsmittel 3 eine bevorzugte Richtung zu geben.

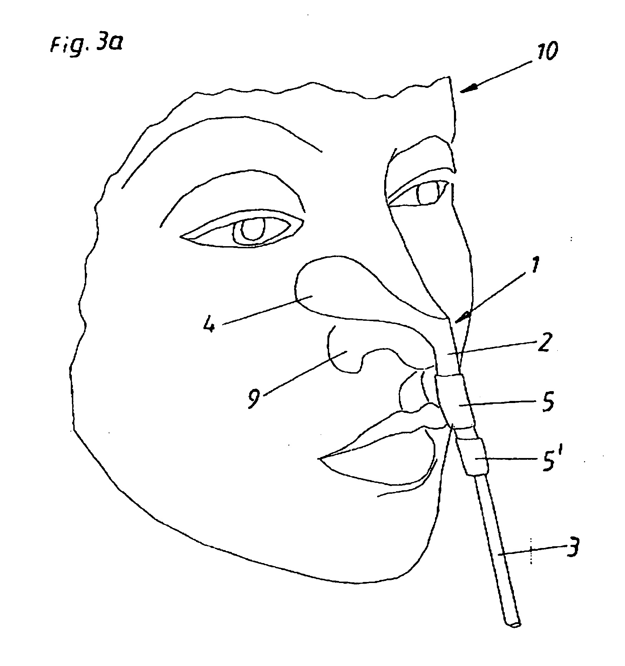

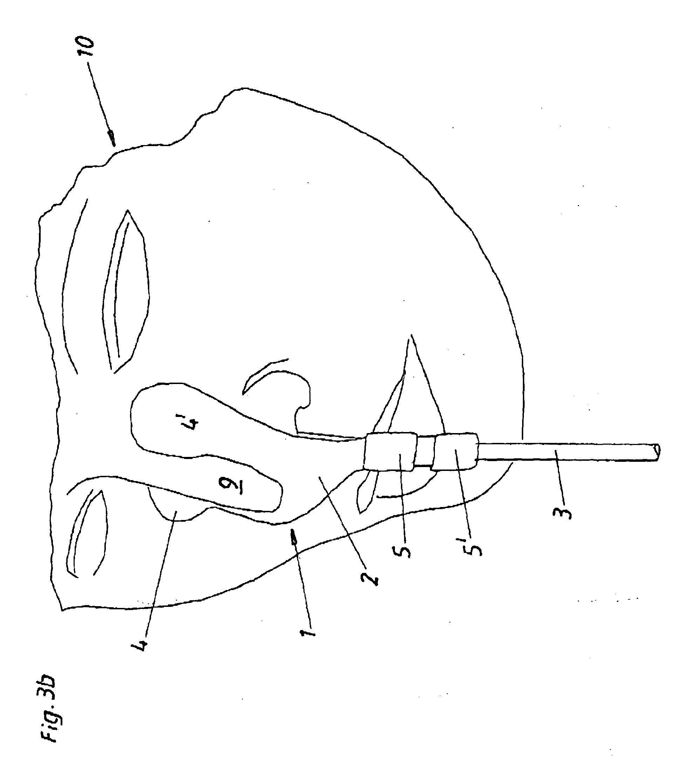

Dazu wird, wie aus Fig. 3a und 3b ersichtlich, der längliche Grundkörper 7 des zweiten Abschnittes B am medizinischen Hilfsmittel 3 zur Anlage gebracht und dann das medizinische Hilfsmittel 3 mittels der Fixierelemente 4, 4', die in Bezug auf den länglichen Grundkörper 7 des zweiten Abschnittes B im Wesentlichen normal und auf gegenüberliegenden Seiten angeordnet sind, fixiert.

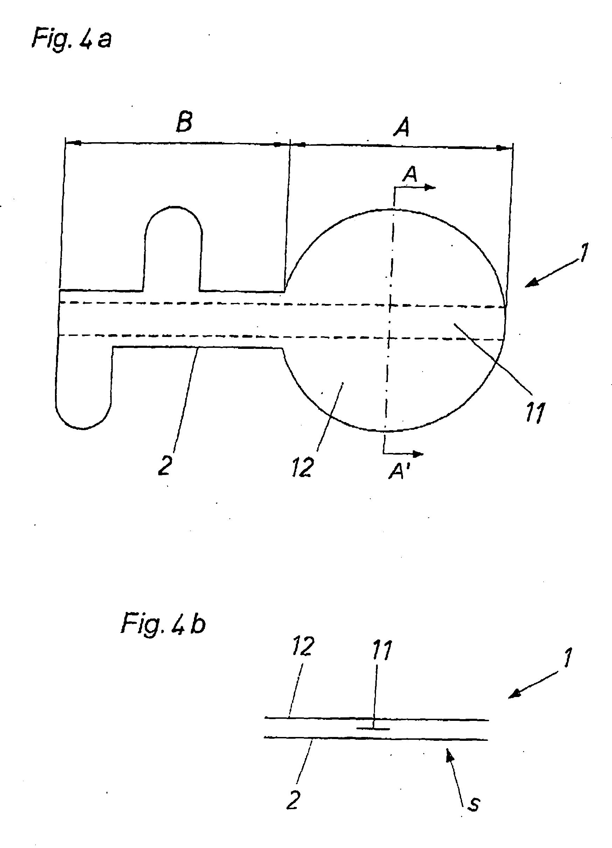

Die in den Fig. 4a und 4b dargestellte Fixiervorrichtung 1 weist ein Trägerelement 2 auf, das von einer einseitig klebenden, transparenten PET-Folie gebildet ist. Das flexible Trägerelement 2, weiches auch aus PE-Schaum oder einem Fliess hergestellt sein könnte, verfügt über einen der Haut der Patienten zuordenbaren ersten Abschnitt A und einen dem medizinischen Hilfsmittel zuordenbaren zweiten Abschnitt B. Bei diesem Ausführungsbeispiel wird das Trägerelement 2 zur Gänze von einer Abdeckung 12 abgedeckt, wobei zwischen der Abdeckung 12 und dem Trägerelement 2 das Versteifungselement 11 angeordnet ist. Die Fixiervorrichtung 1 weist also, wie aus Fig. 4b ersichtlich, - von unten nach oben - ein einseitig klebendes, vorzugsweise transparentes Trägerelement 2, ein Versteifungselement 11 sowie eine einseitig klebende, vorzugsweise transparente, Abdeckung 12 auf. Dabei ist das Versteifungselement zwischen der der klebenden Seite S des Trägerelementes 2 abgewandten Seite des Trägerelementes 2 und der selbstklebenden Abdeckung 12 einlaminiert. Das Versteifungselement 11 wird beim gezeigten Ausführungsbeispiel von einer etwa 190 µm dicken PE-Folie gebildet und ist somit plastisch verformbar. Es versteht sich von selbst, dass auch andere Materialien zur Ausbildung des Versteifungselementes 11 geeignet sind. So könnte das Versteifungselement 11 beispielsweise rückfedemd ausgebildet sein.

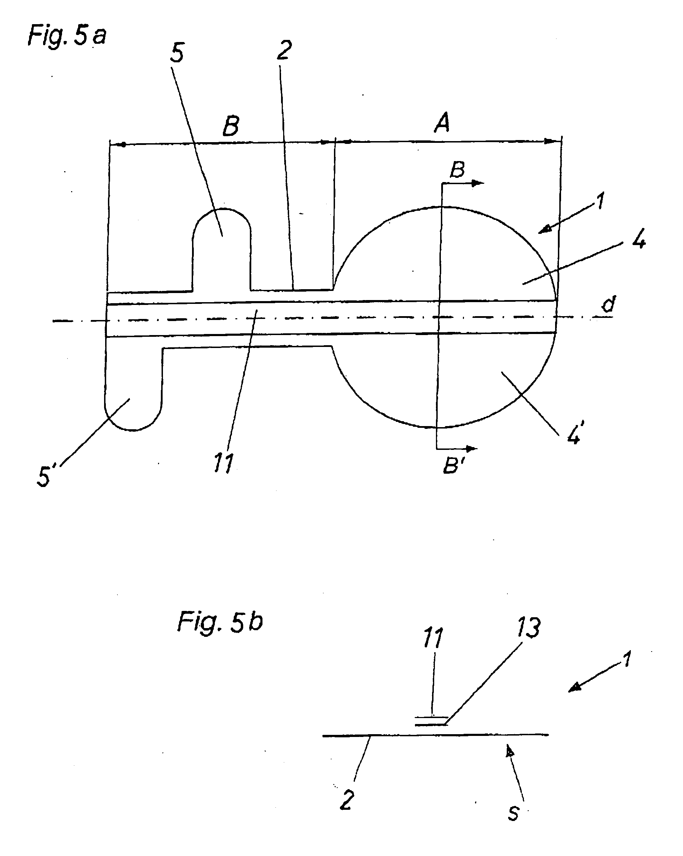

Das in Fig. 5a dargestellte Ausführungsbeispiel einer erfindungsgemäßen Fixiervorrichtung 1 unterscheidet sich von dem in Fig. 4a und 4b dargestellten Ausführungsbeispiel in der Befestigungsart des Versteifungselementes 11 auf dem Trägerelement 2. Wie insbesondere aus Fig. 5b ersichtlich, ist bei diesem Ausführungsbeispiel das Versteifungselement 11 mittels eines doppelseitigen Klebebandes 13 auf der der klebenden Seite S des Trägerelementes 2 gegenüberliegenden Seite des Trägerelementes 2 angeordnet. Dabei ist das Versteifungselement 11 im Wesentlichen entlang der Mittellängsachse d der Fixiervorrichtung 1 am Trägerelement 2 angeordnet. Aus Fig. 5a ist weiters ersichtlich, dass das Trägerelement 2 in seinem ersten Abschnitt A, zwei halbkreisförmige, sich in Bezug auf die Mittellängsachse d gegenüberliegende Fixierelemente 4, 4' aufweist, mit denen die Fixiervorrichtung 1 an der Haut des Patienten befestigt wird. Im zweiten, dem Hilfsmittel zugeordneten Abschnitt B weist das Trägerelement 2 zwei sich in Bezug auf die Mittellängsachse d gegenüberliegende, in Längsrichtung der Fixiervorrichtung 1 gegeneinander versetzte Fixiermittel 5, 5' auf, wobei die Befestigung der Fixiervorrichtung 1 am medizinischen Hilfsmittel mittels dieser Fixiermittel 5, 5`, die um das medizinische Hilfsmittel herum geschlungen werden, erfolgt.

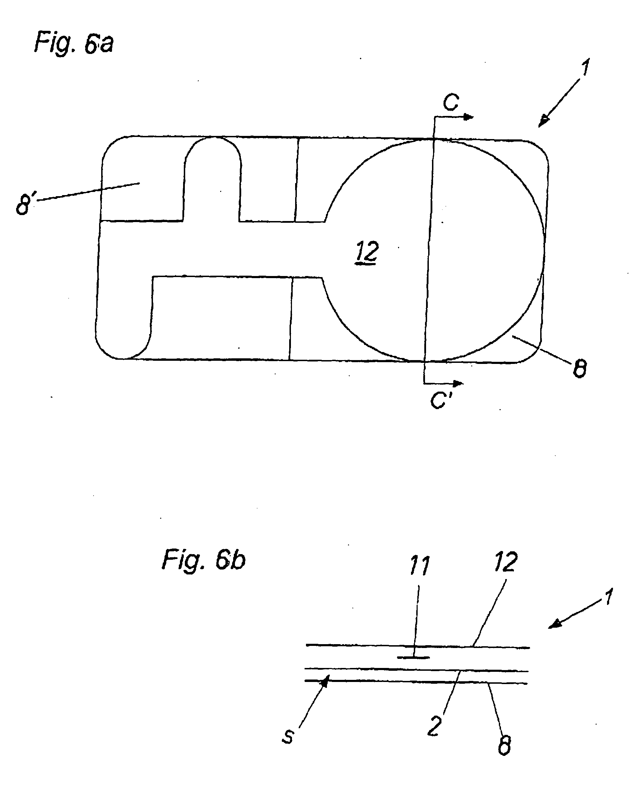

Die Fig. 6a und 6b zeigen das Ausführungsbeispiel aus den Fig. 4a und 4b, wobei die klebende Seite S des Trägerelementes 2 mit einer vorzugsweise silikonisierten Folie 8, 8' abgedeckt ist. Die zweiteilige Ausbildung dieser Folie 8, 8' soll das Ablösen der Folie vom Trägerelement 2 erleichtern.

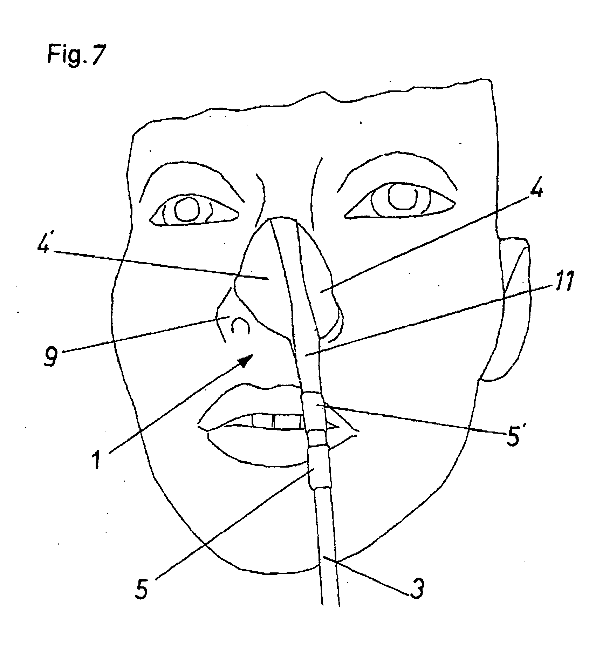

Fig. 7 zeigt ein Anwendungsbeispiel der erfindungsgemäßen Fixiervorrichtung 1. Dabei dient die Fixiervorrichtung 1 der Sicherung eines über die Nase 9 eingeführten, von einer Sonde gebildeten medizinischen Hilfsmittels 3. Eine der gefährlichsten Komplikationen bei der Intubation ist die Dislokation oder das Herausrutschen der Sonde. Um dies zu verhindern, muss eine stabile Verbindung zwischen der Sonde und dem Nasenrücken sichergestellt werden. Dazu wird die Nase nach der Sondenlegung entfettet, um eine gute Klebrigkeit zu gewährleisten. Anschließend wird der erste Teil 8 der Folie von der Fixiervorrichtung 1 abgezogen, sodass die halbkreisförmigen Fixierelemente 4, 4' des Trägerelementes 2 auf der Nase 9 platziert und durch leichtes Andrücken fixiert werden können. Danach wird der zweite Teil 8' der Folie abgezogen und die beiden abstehenden Fixiermittel 5, 5' um die Sonde geschlungen. Durch das einlaminierte Verbindungselement 11 wird verhindert, dass die Sonde in der Nase 9 zuviel Bewegungsfreiheit hat, wodurch es zu Scheuerstellen in der Nase kommen könnte.

Mit der erfindungsgemäßen Fixiervorrichtung wird also erreicht, dass einerseits eine mögliche Beeinträchtigung an der Nase gering gehalten wird, aber auch die Beeinträchtigung des Patienten durch die Fixierung minimiert ist. Die erfindungsgemäße Fixiervorrichtung zeichnet sich weiters durch eine sehr einfache Handhabung und eine sehr wirkungsvolle Fixierung des Hilfsmittels bei geringer Beeinträchtigung des Patienten aus.

Die dargestellten Ausführungsbeispiele von Fixiervorrichtungen für medizinische Hilfsmittel sind selbstverständlich nicht in einschränkendem Sinne zu verstehen, sondern eben nur einzelne Beispiele von zahlreichen Möglichkeiten, den Erfindungsgedanken einer Fixiervorrichtung für medizinische Hilfsmittel zu verwirklichen.

## Patentansprüche

1. Fixiervorrichtung zum Befestigen eines in eine Körperöffnung einbringbaren medizinischen Hilfsmittels, insbesondere durch die Nase einbringbare Beatmungstuben, an der Haut eines Subjektes, mit einem Trägerelement, das wenigstens einen ersten dem Subjekt zuordenbaren und einen zweiten dem Hilfsmittel zuordenbaren Abschnitt aufweist, die zumindest bereichsweise einseitig klebend ausgebildet sind, wobei der zweite dem medizinischen Hilfsmittel zuordenbare Abschnitt (B) einen länglichen Grundkörper (7) und wenigstens zwei in Bezug auf die Längsachse des Grundkörpers (7) gegenüberliegende, im Wesentlichen normal zur Längsachse angeordnete Fixiermittel (5,5') aufweist, **dadurch gekennzeichnet, dass** die Fixiermittel (5,5') in Längsrichtung des Grundskörpers (7) voneinander beabstanded sind.

2. Fixiervorrichtung nach Anspruch 1, **dadurch gekennzeichnet, dass** ein Fixiermittel (5') am freien Ende des Grundkörpers (7) angeordnet ist.

3. Fixiervorrichtung nach Anspruch 1 oder 2, **dadurch gekennzeichnet, dass** die wenigstens zwei Fixiermittel (5,5') zumindest bereichsweise einseitig klebend ausgebildet sind.

4. Fixiervorrichtung nach einem der Ansprüche 1 bis 3, **dadurch gekennzeichnet, dass** der erste dem Subjekt zuordenbare Abschnitt (A) des Trägerelementes (2) wenigstens zwei voneinander unabhängig am Subjekt befestigbare Fixierelemente (4,4') aufweist.

5. Fixiervorrichtung nach Anspruch 4, **dadurch gekennzeichnet, dass** der erste Abschnitt (A) v-förmig ausgebildet ist bzw. die Fixierelemente (4,4') des ersten Abschnittes (A) v-förmig angeordnet sind.

6. Fixiervorrichtung nach Anspruch 4 oder 5, **dadurch gekennzeichnet, dass** wenigstens eines der beiden Fixierelemente (4,4'), vorzugsweise beide Fixierelemente (4,4'), einen im Wesentlichen länglichen Grundkörper (6.6') und einen gegenüber dem Grundköper (6,6') verbreiterten, vorzugsweise kreisflächenförmig oder ellipsenförmig ausgebildeten Endabschnitt (C,C') aufweist.

7. Fixiervorrichtung nach Anspruch 6, **dadurch gekennzeichnet, dass** die Fixierelemente (4,4') zumindest bereichsweise einseitig klebend ausgebildet sind.

8. Fixiervorrichtung nach einem der Ansprüche 1 bis 7, **dadurch gekennzeichnet, dass** die Fixiervorrichtung (1) wenigstens ein mit dem Trägerelement (2) vorzugsweise unlösbar verbundenes Versteifungselement (11) aufweist.

9. Fixiervorrichtung nach Anspruch 8, **dadurch gekennzeichnet, dass** das wenigstens eine Versteifungselement (11) auf der der klebenden Seite (S) gegenüberliegenden Seite des Trägerelementes (2), das Trägerelement (2) vorzugsweise teilweise abdeckend, angeordnet ist.

10. Fixiervorrichtung nach Anspruch 8 oder 9, **dadurch gekennzeichnet, dass** das Trägerelement (2) einen ersten dem Patienten zuordenbaren Abschnitt (A) und einen zweiten dem Hilfsmittel (3) zuordenbaren Abschnitt (B) aufweist, wobei das Versteifungselement (11) den ersten und den zweiten Abschnitt (A, B) zumindest teilweise überdeckend auf dem Trägerelement (2) angeordnet ist.

11. Fixiervorrichtung nach einem der Ansprüche 8 bis 10, **dadurch gekennzeichnet, dass** das Trägerelement (2) zweiteilig ausgebildet ist.

12. Fixiervorrichtung nach einem der Ansprüche 8 bis 11, **dadurch gekennzeichnet, dass** das Versteifungselement (11) auf das Trägerelement (2) geklebt ist, vorzugsweise mittels eines doppelseitigen Klebebandes (13).

13. Fixiervorrichtung nach einem der Ansprüche 8 bis 12, **dadurch gekennzeichnet, dass** auf der der klebenden Seite (S) gegenüber liegenden Seite des Trägerelementes (2) eine Abdeckung (12) angeordnet ist, wobei das Versteifungselement (11) zwischen dem Trägerelement (2) und der Abdeckung (12) eingeschlossen, vorzugsweise einlaminiert, ist.

14. Fixiervorrichtung nach Anspruch 13, **dadurch gekennzeichnet, dass** die Abdeckung (12) von einer einseitig klebenden Folie gebildet ist.

15. Fixiervorrichtung nach Anspruch 13 oder 14, **dadurch gekennzeichnet, dass** die Abdeckung (12) eine vorzugsweise transparente PET-Folie ist.

16. Fixiervorrichtung nach einem der Ansprüche 8 bis 15, **dadurch gekennzeichnet,** das vorzugsweise streifenförmige Versteifungselement (11) im Wesentlichen entlang der Mittellängsachse (d) des Trägerelementes (2) an diesem angeordnet ist.

17. Fixiervorrichtung nach einem der Ansprüche 8 bis 16, **dadurch gekennzeichnet, dass** das Versteifungselement (11) von einer zwischen 100 µm und 300 µm, vorzugsweise zwischen 150 µm und 250 µm, dicken PE-Folie gebildet ist.

18. Fixiervorrichtung nach Anspruch 17, **dadurch gekennzeichnet, dass** die PE-Folie etwa 190 µm dick ist.

19. Fixiervorrichtung nach einem der Ansprüche 1 bis 18, **dadurch gekennzeichnet, dass** das Trägerelement (2) einseitig, vorzugsweise vollflächig, klebend ausgebildet ist.

20. Fixiervorrichtung nach Anspruch 19, **dadurch gekennzeichnet, dass** das Trägerelement (2) von einer Selbstklebefolie, vorzugsweise einer einseitig klebenden Polymer-Folie, gebildet ist.

21. Fixiervorrichtung nach einem der Ansprüche 1 bis 20, **dadurch gekennzeichnet, dass** das Trägerelement (2) von einer transparenten, selbstklebenden PET-Folie gebildet ist.

22. Fixiervorrichtung nach einem der Ansprüche 1 bis 21, **dadurch gekennzeichnet, dass** zumindest die klebenden Bereiche des Trägerelementes (2) mit einer vorzugsweise silikonisierten Abdeckung (8, 8') versehen sind.

## Claims

1. A fixing device for fixing a medical aid which can be introduced into a body orifice, in particular breathing tubes which can be introduced through the nose, to the skin of a subject, comprising a carrier element which has at least a first portion which can be associated with the subject and a second portion which can be associated with the aid, which portions are at least region-wise adhesive on one side, wherein the second portion (B) which can be associated with the medical aid has an elongate main body (7) and at least two fixing means (5, 5') which are disposed in opposite relationship with respect to the longitudinal axis of the main body (7) and which are arranged substantially normal to the longitudinal axis **characterised in that** the fixing means (5, 5') are spaced from each other in the longitudinal direction of the main body (7).

2. A fixing device according to claim 1 **characterised in that** a fixing means (5') is arranged at the free end of the main body (7).

3. A fixing device according to claim 1 or claim 2 **characterised in that** the at least two fixing means (5, 5') are at least region-wise adhesive on one side.

4. A fixing device according to one of claims 1 to 3 **characterised in that** the first portion (A) of the carrier element (2), that can be associated with the subject, has at least two fixing elements (4, 4') which can be fixed independently of each other to the subj ect.

5. A fixing device according to claim 4 **characterised in that** the first portion (A) is of a v-shaped configuration or the fixing elements (4, 4') of the first portion (A) are arranged in a v-shape.

6. A fixing device according to claim 4 or claim 5 **characterised in that** at least one of the two fixing elements (4, 4') and preferably both fixing elements (4, 4') has or have a substantially elongate main body (6, 6') and an end portion (C, C') which is widened in relation to the main body (6, 6') and which is preferably in the shape of a circular surface or in the form of an ellipse.

7. A fixing device according to claim 6 **characterised in that** the fixing elements (4, 4') are at least region-wise adhesive on one side.

8. A fixing device according to one of claims 1 to 6 **characterised in that** the fixing device (1) has at least one stiffening element (11) which is preferably non-releasably connected to the carrier element (2).

9. A fixing device according to claim 8 **characterised in that** the at least one stiffening element (11) is arranged on the side of the carrier element (2), that is opposite to the adhesive side (S), preferably partially covering over the carrier element (2).

10. A fixing device according to claim 8 or claim 9 **characterised in that** the carrier element (2) has a first portion (A) which can be associated with the patient and a second portion (B) which can be associated with the aid (3), wherein the stiffening element (11) is arranged on the carrier element (2) at least partially covering over the first and second portions (A, B).

11. A fixing device according to one of claims 8 to 10 **characterised in that** the carrier element (2) is of a two-part configuration.

12. A fixing device according to one of claims 8 to 11 **characterised in that** the stiffening element (11) is glued on the carrier element (2), preferably by means of a double-sided adhesive tape (13).

13. A fixing device according to one of claims 8 to 12 **characterised in that** a cover means (12) is arranged on the side of the carrier element (2), that is opposite to the adhesive side (S), wherein the stiffening element (11) is enclosed, preferably by being laminated, between the carrier element (2) and the cover means (12).

14. A fixing device according to claim 13 **characterised in that** the cover means (12) is formed by a film which is adhesive on one side.

15. A fixing device according to claim 13 or claim 14 **characterised in that** the cover means (12) is a preferably transparent PET film.

16. A fixing device according to one of claims 8 to 15 **characterised in that** the preferably strip-shaped stiffening element (11) is arranged on the carrier element (2) substantially along the longitudinal centre line (d) thereof.

17. A fixing device according to one of claims 8 to 16 **characterised in that** the stiffening element (11) is formed by a PE film which is between 100 µm and 300 µm, preferably between 150 µm and 250 µm, in thickness.

18. A fixing device according to claim 17 **characterised in that** the PE film is about 190 µm in thickness.

19. A fixing device according to one of claims 1 to 18 **characterised in that** the carrier element (2) is adhesive on one side, preferably over its entire area.

20. A fixing device according to claim 19 **characterised in that** the carrier element (2) is formed by a self-adhesive film, preferably a polymer film which is adhesive on one side.

21. A fixing device according to one of claims 1 to 20 **characterised in that** the carrier element (2) is formed by a transparent, self-adhesive PET film.

22. A fixing device according to one of claims 1 to 21 **characterised in that** at least the adhesive regions of the carrier element (2) are provided with a preferably siliconised cover means (8, 8').

## Revendications

1. Dispositif de fixation pour la mise en place sur la peau d'un patient d'un accessoire médical applicable dans un orifice corporel, en particulier de tubes respiratoires introduits par le nez, avec un élément de support comportant au moins une première partie côté patient et une deuxième partie côté accessoire, lesquelles sont au moins partiellement adhésives sur une face, la deuxième partie (B) côté accessoire médical comportant un corps de base oblong (7) et au moins deux moyens de fixation (5, 5') opposés relativement à l'axe longitudinal du corps de base (7), disposés sensiblement perpendiculairement à l'axe longitudinal, **caractérisé en ce que** les moyens de fixation (5, 5') sont espacés l'un de l'autre dans la direction longitudinale du corps de base (7).

2. Dispositif de fixation selon la revendication 1, **caractérisé en ce qu'**un moyen de fixation (5') est disposé sur l'extrémité libre du corps de base (7).

3. Dispositif de fixation selon la revendication 1 ou la revendication 2, **caractérisé en ce que** les au moins deux moyens de fixation (5, 5') sont au moins partiellement adhésifs sur une face.

4. Dispositif de fixation selon l'une des revendications 1 à 3, **caractérisé en ce que** la première partie (A) côté patient de l'élément de support (2) comporte au moins deux éléments de fixation (4, 4') pouvant être mis en place sur le patient indépendamment l'un de l'autre.

5. Dispositif de fixation selon la revendication 4, **caractérisé en ce que** la première partie (A) est en forme de V, ou que les éléments de fixation (4, 4') de la première partie (A) sont disposés en V.

6. Dispositif de fixation selon la revendication 4 ou la revendication 5, **caractérisé en ce qu'**au moins un des deux éléments de fixation (4, 4'), de préférence les deux éléments de fixation (4, 4'), comporte un corps de base (6, 6') sensiblement oblong, et une partie d'extrémité (C, C') élargie par rapport au corps de base (6, 6'), de préférence de forme circulaire ou ellipsoïdale.

7. Dispositif de fixation selon la revendication 6, **caractérisé en ce que** les éléments de fixation (4, 4') sont au moins partiellement adhésifs sur une face.

8. Dispositif de fixation selon l'une des revendications 1 à 7, **caractérisé en ce que** le dispositif de fixation (1) comporte au moins un élément de renforcement (11) de préférence fixement raccordé à l'élément de support (2).

9. Dispositif de fixation selon la revendication 8, **caractérisé en ce que** le ou les éléments de renforcement (11) est disposé sur la face de l'élément de support (2) opposée à la face adhésive (8), en recouvrant de préférence au moins partiellement l'élément de support (2).

10. Dispositif de fixation selon la revendication 8 ou la revendication 9, **caractérisé en ce que** l'élément de support (2) comporte une première partie (A) côté patient et une deuxième partie (B) côté accessoire (3), l'élément de renforcement (11) étant disposé sur l'élément de support (2) en recouvrant au moins partiellement la première et la deuxième parties (A, B).

11. Dispositif de fixation selon l'une des revendications 8 à 10, **caractérisé en ce que** l'élément de support (2) est réalisé en deux parties.

12. Dispositif de fixation selon l'une des revendications 8 à 11, **caractérisé en ce que** l'élément de renforcement (11) est collé sur l'élément de support (2), de préférence au moyen d'un ruban adhésif double-face (13) .

13. Dispositif de fixation selon l'une des revendications 8 à 12, **caractérisé en ce qu'**une couverture (12) est disposée sur la face de l'élément de support (2) opposée à la face adhésive (8), l'élément de renforcement (11) étant inclus, de préférence par stratification, entre l'élément de support (2) et la couverture (12).

14. Dispositif de fixation selon la revendication 13, **caractérisé en ce que** la couverture (12) est formée par un film adhésif sur une face.

15. Dispositif de fixation selon la revendication 13 ou la revendication 14, **caractérisé en ce que** la couverture (12) est un film PET de préférence transparent.

16. Dispositif de fixation selon l'une des revendications 8 à 15, **caractérisé en ce que** l'élément de renforcement (11) de préférence en forme de bande est sensiblement disposé le long de l'axe longitudinal médian (d) de l'élément de support (2) et contre celui-ci.

17. Dispositif de fixation selon l'une des revendications 8 à 16, **caractérisé en ce que** l'élément de renforcement (11) est formé par un film PE d'épaisseur comprise entre 100 µm et 300 µm, de préférence entre 150 µm et 250 µm.

18. Dispositif de fixation selon la revendication 17, **caractérisé en ce que** l'épaisseur du film PE est sensiblement de 190 µm.

19. Dispositif de fixation selon l'une des revendications 1 à 18, **caractérisé en ce que** l'élément de support (2) est adhésif sur une face, de préférence sur toute celle-ci.

20. Dispositif de fixation selon la revendication 19, **caractérisé en ce que** l'élément de support (2) est formé par un film autocollant, de préférence un film polymère adhésif sur une face.

21. Dispositif de fixation selon l'une des revendications 1 à 20, **caractérisé en ce que** l'élément de support (2) est formé par un film PET transparent autocollant.

22. Dispositif de fixation selon l'une des revendications 1 à 21, **caractérisé en ce qu'**au moins les zones adhésives de l'élément de support (2) sont pourvues d'une couverture (8, 8') de préférence siliconisée.
